# EUROPEAN PATENT APPLICATION

(11) **EP 4 603 574 A1**
(43) Date of publication of application: **20.08.2025**
(21) Application number: 22962191.7
(22) Date of filing: 22.11.2022
(51) Int. Cl.: C12N 1/20, A61K 35/74, A61P 3/00, A61P 9/00, A61P 13/12, A23L 33/135, A23K 10/16, C12R 1/01

(54) **PARACOCCUS AMINOVORANS BM109 STRAIN AND USES THEREOF**

(30) Priority: 11.10.2022 KR 20220129834
(71) Applicant: Biome Inc., Seodaemun-gu Seoul 03722 (KR)
(72) Inventor: KIM, Jun Beom, Seoul 03727 (KR); SEO, Mu Kyung, Seoul 08735 (KR); HONG, Min Young, Seoul 02814 (KR)
(74) Representative: Müller Schupfner & Partner Patent- und Rechtsanwaltspartnerschaft mbB (Muc)
(86) International application number: PCT/KR2022/018514
(87) International publication number: WO 2024/080441

(57) **Abstract**

The present invention provides a *Paracoccus aminovorans* BM109 strain (Accession number KCTC 15059BP) having excellent removal of trimethylamine (TMA) or trimethylamine N-oxide (TMAO), and a pharmaceutical composition for preventing or treating diseases caused by TMA or TMAO, a health functional food composition for preventing or improving diseases caused by TMA or TMAO, and a feed composition for preventing or improving diseases caused by TMA or TMAO, including the strain as an active ingredient.

## Description

### [Technical Field]

The present invention relates to *a Paracoccus aminovorans* BM109 strain and a use thereof.

### [Background Art]

Dietary choline, carnitine, betaine, etc. from egg yolk, soy, high-fat dairy products, and meat consumed by mammals are metabolized into trimethylamine (TMA) by microflora in the gut. Then, TMA is absorbed into the blood by passive diffusion through cell membranes in the gut, and the absorbed TMA is converted to trimethylamine N-oxide (TMAO) by flavin-containing monooxygenase-3 (FMO3) in the liver and secreted into urine.

High concentration of TMAO produced from dietary choline, carnitine, betaine, etc. plays a role in cholesterol metabolism, vascular inflammation, and the formation of unstable plaques on artery walls, and furthermore, may cause cardiovascular diseases including atherosclerosis and heart failure. The TMAO may also cause kidney diseases, including glomerulonephritis and IgA nephropathy.

Meanwhile, patients with trimethylaminuria having an autosomal recessive mutation in an FMO3 gene cannot convert TMA to TMAO, and the TMA accumulated in the body is released through sweat, urine, or breathing, which may cause a fish odor syndrome, which causes a characteristic odor such as fishy odor. There is currently no known treatment for trimethylaminuria other than avoiding foods containing TMA and its precursors (e.g., choline, lecithin, and TMAO), such as fish, eggs, beans, soy products, and red meat.

Therefore, there is a need for the development of substances effective in preventing or treating diseases caused by TMA or TMAO accumulation, including trimethylaminuria.

The background art of the invention has been prepared to further facilitate understanding of the present invention. It should not be understood that the matters described in the background art of the invention exist as prior arts.

### [Detailed Description of the Invention]

### [Technical Problems]

Meanwhile, the present inventors noted that a microbial strain that was able to inhabit the gut and excellent in removing trimethylamine (TMA) or trimethylamine N-oxide (TMAO) may be an effective method for treating TMA or TMAO accumulated at a high concentration in the body.

Accordingly, the present inventors attempted to develop a microbial strain that was able to inhabit the gut and excellent in removing TMA or TMAO, and a pharmaceutical composition including the microbial strain.

More specifically, the present inventors noted that a *Paracoccus aminovorans* BM109 strain (Accession number KCTC 15059BP) was able to inhabit the gut and had enzymes required for removing TMA or TMAO and had an excellent effect of removing TMA or TMAO compared to other strains.

As a result, the present inventors found that the *Paracoccus aminovorans* BM109 strain of the present invention had trimethylamine N-oxide (TMAO) reductase, which was not inherently present in *Paracoccus aminovorans* strains belonging to the same species, and thus had an excellent effect of removing TMA or TMAO compared to *Paracoccus aminovorans* strains belonging to the same species.

Therefore, an object of the present invention is to provide a *Paracoccus aminovorans* BM109 (Accession number KCTC 15059BP) strain having an effect of removing TMA or TMAO.

Another object of the present invention is to provide a composition for preventing or treating diseases caused by TMA or TMAO including a *Paracoccus aminovorans* BM109 (Accession number KCTC 15059BP) strain having an effect of removing TMA or TMAO.

The objects of the present invention are not limited to the aforementioned objects, and other objects, which are not mentioned above, will be apparent to those skilled in the art from the following description.

### [Technical Solution]

An aspect of the present invention provides *a Paracoccus aminovorans* BM109 (Accession number KCTC 15059BP) strain having the activity of removing TMA or TMAO.

According to a feature of the present invention, the *Paracoccus aminovorans* BM109 strain may have excellent TMA or TMAO degradation ability.

According to another feature of the present invention, the *Paracoccus aminovorans* BM109 strain may further include a trimethylamine (TMA) monooxygenase gene represented by SEQ ID NO: 1.

According to yet another feature of the present invention, the *Paracoccus minovorans* BM109 strain may include a trimethylamine N-oxide (TMAO) reductase gene represented by SEQ ID NO: 2.

According to yet another feature of the present invention, the *Paracoccus aminovorans* BM109 strain may further include a trimethylamine (TMA) dehydrogenase 1 gene represented by SEQ ID NO: 3.

According to yet another feature of the present invention, the *Paracoccus aminovorans* BM109 strain may further include a TMA dehydrogenase 2 gene represented by SEQ ID NO: 4.

According to yet another feature of the present invention, the *Paracoccus aminovorans* BM109 strain may further include a formaldehyde dehydrogenase gene represented by SEQ ID NO: 5.

According to yet another feature of the present invention, the *Paracoccus aminovorans* BM109 strain may further include a formate dehydrogenase 1 gene represented by SEQ ID NO: 6.

According to yet another feature of the present invention, the *Paracoccus aminovorans* BM109 strain may further include a formate dehydrogenase 2 gene represented by SEQ ID NO: 11, consisting of a set of four separate gene sequences represented by SEQ ID NOs: 7 to 10.

According to yet another feature of the present invention, the *Paracoccus aminovorans* BM109 strain characterized in that the strainincludes a TMAO reductase gene represented by SEQ ID NO: 2 of converting trimethylamine N-oxide (TMAO) to trimethylamine (TMA), thereby having further increased the TMA or TMAO removal ability, compared to *Paracoccus aminovorans* strains without the TMAO reductase.

Furthermore, the *Paracoccus aminovorans* BM109 strain of the present invention has all of a TMA dehydrogenase 1 gene represented by SEQ ID NO: 3 and a TMA dehydrogenase 2 gene represented by SEQ ID NO: 4, which degrade TMA, a formaldehyde dehydrogenase gene represented by SEQ ID NO: 5, which degrades formaldehyde into formate, and a formate dehydrogenase gene 1 represented by SEQ ID NO: 6 and a formate dehydrogenase gene 2 represented by SEQ ID NO: 11, which ultimately degrade formate into carbon dioxide, so as to completely degrade TMA and TMAO into carbon dioxide as a final product via formaldehyde and formate.

According to yet another feature of the present invention, the *Paracoccus aminovorans* BM109 strain may have increased trimethylamine removal ability under anaerobic conditions.

In an embodiment of the present invention, the anaerobic conditions may be similar to an environment in the gut, with an oxygen concentration of 2 to 12%, more preferably 3 to 10%.

Yet another aspect of the present invention provides a pharmaceutical composition for preventing or treating diseases caused by TMA or TMAO, including *a Paracoccus aminovorans* BM109 strain, live cells thereof, dead cells thereof, a freeze-dried product thereof, a lysate thereof, an extract thereof, or a compound thereof.

According to an embodiment of the present invention, the disease caused by TMA or TMAO may be a metabolic disease, and the metabolic disease may be trimethylaminuria or fish odor syndrome.

In addition, in an embodiment of the present invention, the disease caused by TMA or TMAO may be a cardiovascular disease caused by accumulation of TMAO, and the cardiovascular disease may be at least one selected from atherosclerosis, severe heart failure, coronary heart disease, inflammatory heart disease, valvular heart disease, stroke, thrombosis, myocardial infarction, and reactive thrombocytosis.

In addition, in an embodiment of the present invention, the disease caused by TMA or TMAO may be a kidney disease caused by accumulation of TMAO, and the kidney disease may be at least one selected from renal failure, chronic kidney disease, polycystic kidney disease, glomerulonephritis, IgA nephropathy, and kidney syndrome.

Yet another aspect of the present invention provides a health functional food composition for preventing or improving diseases caused by TMA or TMAO, including a *Paracoccus aminovorans* BM109 strain, live cells thereof, dead cells thereof, a freeze-dried product thereof, a lysate thereof, an extract thereof, or a compound thereof.

Yet another aspect of the present invention provides a feed composition for preventing or improving diseases caused by TMA or TMAO, including a *Paracoccus aminovorans* BM109 strain, live cells thereof, dead cells thereof, a freeze-dried product thereof, a lysate thereof, an extract thereof, or a compound thereof.

### [Effects of the Invention]

According to the present invention, it is possible to provide a *Paracoccus aminovorans* BM109 (Accession number KCTC 15059BP) strain having an effect of removing TMA or TMAO.

Further, it is possible to provide a pharmaceutical composition for preventing or treating diseases caused by TMA or TMAO including a *Paracoccus aminovorans* BM109 (Accession number KCTC 15059BP) strain having an effect of removing TMA or TMAO.

Accordingly, according to the present invention, it is possible to degrade TMA or TMAO in the body, more specifically in the gut or blood, to be converted into a substance harmless to the human body, and thus may have the effect of preventing and treating diseases caused by TMA or TMAO.

More specifically, the *Paracoccus aminovorans* BM109 strain of the present invention can survive and proliferate in the gut of the subject, and thus may increase a period having a TMA or TMAO degradation effect. Therefore, the *Paracoccus aminovorans* BM109 strain of the present invention has an excellent effect of preventing and treating diseases caused by TMA or TMAO.

In addition, the *Paracoccus aminovorans* BM109 strain of the present invention is not a recombinant microorganism, that is, a genetically modified organism (GMO), and thus does not have any unknown harmful effect of GMOs.

Furthermore, the strain of the present invention is a microorganism derived from human feces and may survive and proliferate under both *in vitro* aerobic and anaerobic conditions, and thus can also be used as a composition for controlling the ecological environment, such as removing unpleasant odor caused by TMA.

The effects of the present invention are not limited by the foregoing, and other various effects are anticipated herein.

### [Brief Description of Drawings]

FIG. 1 illustrates enzymes related to TMA and TMAO degradation of a *Paracoccus aminovorans* BM109 strain used in various embodiments of the present invention and a TMA and TMAO metabolic pathway using the enzymes.
FIG. 2 illustrates results of identifying enzyme genes related to TMA and TMAO degradation of a *Paracoccus aminovorans* BM109 strain used in various embodiments of the present invention.
FIG. 3 illustrates *in vitro* test results for TMA and TMAO degradation ability of a *Paracoccus aminovorans* BM109 strain used in various embodiments of the present invention.
FIG. 4 illustrates results of concentrations of TMA and TMAO in feces from *in vivo* tests for TMA and TMAO degradation ability of a *Paracoccus aminovorans* BM109 strain used in various embodiments of the present invention.
FIG. 5 illustrates results of concentrations of TMA and TMAO in blood from *in vivo* tests for TMA and TMAO degradation ability of a *Paracoccus aminovorans* BM109 strain used in various embodiments of the present invention.

### [Best Modes for Carryng out the Invention]

Advantages and features of the present invention, and methods for accomplishing the same will be more clearly understood from embodiments to be described below in detail with reference to the accompanying drawings. However, the present invention is not limited to the embodiments set forth below and will be embodied in various different forms. The embodiments are just for rendering the disclosure of the present invention complete and are set forth to provide a complete understanding of the scope of the invention to those skilled in the art to which the present invention pertains, and the present invention will only be defined by the scope of the claims.

Hereinafter, terms used in the present specification will be described for clarity of explanation.

As used herein, the term "subject" may mean an animal, including humans, with a disease caused by TMA or TMAO, and may be used interchangeably with terms such as a host, a subject, and a patient.

As used herein, the term "disease caused by TMA or TMAO" may mean a disease caused by the accumulation of TMA or TMAO and may further mean a metabolic disease including trimethylaminuria or fish odor syndrome.

Furthermore, the disease may be a cardiovascular disease including at least one selected from the group consisting of atherosclerosis, severe heart failure, coronary arterial heart disease, inflammatory heart disease, valvular heart disease, stroke, thrombosis, myocardial infarction, and reactive thrombocytosis. In addition, the disease may be a kidney disease including at least one selected from the group consisting of renal failure, chronic kidney disease, polycystic kidney disease, glomerulonephritis, IgA nephropathy, and nephrotic syndrome.

As used herein, the term "aerobic condition" may mean a condition in which the oxygen concentration is 10% (v/v) or more.

As used herein, the term "anaerobic condition" may mean a condition in which the oxygen concentration is 3 to 10% (v/v).

As used herein, the term "gut environment" is limited to the gut oxygen concentration condition and may mean oxygen concentration conditions of 3 to 10% (v/v). Additionally, the gut environment may refer to the gut environment of mammals, including humans.

As used herein, the term "pharmaceutical composition" may mean a composition prepared for preventing or treating diseases.

The *Paracoccus aminovorans* BM109 strain may be included in an amount of about 0.001 wt% to about 90.0 wt% based on the total weight of the composition. For example, the *Paracoccus aminovorans* BM109 strain may be included in an amount of about 0.01 wt% to about 60 wt%, about 0.01 wt% to about 40 wt%, about 0.01 wt% to about 30 wt%, about 0.01 wt% to about 20 wt%, about 0.01 wt% to about 10 wt%, about 0.01 wt% to about 5 wt%, about 0.05 wt% to about 60 wt%, about 0.05 wt% to about 40 wt%, about 0.05 wt% to about 30 wt%, about 0.05 wt% to about 20 wt%, about 0.05 wt% to about 10 wt%, about 0.05 wt% to about 5 wt%, about 0.1 wt% to about 60 wt%, about 0.1 wt% to about 40 wt%, about 0.1 wt% to about 30 wt%, about 0.1 wt% to about 20 wt%, about 0.1 wt% to about 10 wt%, or about 0.1 wt% to about 5 wt%. The composition may be formulated and used in various forms according to conventional methods. For example, the composition may be an oral or eye-drop formulation. The oral formulation may be one or more formulations selected from the group consisting of tablets, granules, pills, capsules, liquid forms, jellies, and gums.

The composition may further include a pharmaceutically acceptable carrier, an excipient, or a diluent. The pharmaceutically acceptable carrier, the excipient, or the diluent may include at least one selected from the group consisting of lactose, dextrose, sucrose, calcium silicate, cellulose, methylcellulose, microcrystalline cellulose, polyvinylpyrrolidone, water, methylhydroxybenzoate, propylhydroxybenzoate, talc, magnesium stearate, and mineral oil, but is not limited thereto.

A pharmaceutically effective amount and an effective dose of the pharmaceutical composition may vary depending on a formulation method, an administration method, an administration time, and/or an administration route of the pharmaceutical composition. Further, the pharmaceutically effective amount and the effective dose may vary depending on various factors including the type and degree of a response to be achieved by administration of the pharmaceutical composition, the type, age, body weight, and general health condition, symptoms or severity of a disease, sex, diet, and excretion of a subject to be administered, drugs used simultaneously or separately for the corresponding subject, other composition ingredients, and the like, and similar factors well-known in the field of medicine. The administration may be performed by methods known in the art. For example, the pharmaceutical composition may be administered directly to a subject by any means through a route such as intravenous, intramuscular, oral, transdermal, mucosal, intranasal, intratracheal or subcutaneous administration. The pharmaceutical composition may be administered systemically or locally. Those skilled in the art may easily determine and prescribe an effective dose for a desired treatment. The pharmaceutical composition according to the present invention may be administered once a day, or several divided times. Accordingly, the dose does not limit the scope of the present invention in any aspect.

As used herein, the term "active ingredient" or "effective amount" may mean any amount of the composition sufficient to alleviate, inhibit the progression of, or prevent a disease, a disorder, a condition, or one or more symptoms thereof.

As used herein, the term "prevention" means all actions that inhibit or delay the symptoms of a disease caused by TMA or TMAO to approach a normal control group by administering the composition including the *Paracoccus aminovorans* BM109 strain according to the present invention.

As used herein, the term "treatment" means all actions that improve or beneficially change the symptoms of a disease caused by TMA or TMAO to be equal or similar to the normal control group by administering the composition including the *Paracoccus aminovorans* BM109 strain according to the present invention.

As used herein, the term "administering" is used interchangeably with "introducing" or "implanting" and may mean disposition of a composition according to an embodiment into a subject by a method or route that causes at least partial localization of the composition according to an embodiment to a desired site. The composition or at least some of the composition ingredients according to an embodiment may be administered by any suitable route to be delivered to a desired location within a living subject.

The administration may be performed by methods known in the art. For example, the pharmaceutical composition may be administered directly to a subject by any means through a route such as intravenous, intramuscular, oral, transdermal, mucosal, intranasal, intratracheal or subcutaneous administration. The pharmaceutical composition may be administered systemically or locally. Preferably, the pharmaceutical composition may be orally administered.

The subject may be mammals. The mammal may be human, dog, cat, cow, goat, or pig.

As used herein, the term "improvement of disease caused by TMA or TMAO" may mean alleviating the symptoms of an existing disease.

As used herein, the term "health functional food" may be in the form of drinks, meat, sausage, bread, biscuits, rice cake, chocolate, candies, snacks, confectionery, pizza, ramen, other noodles, gum, dairy products including ice cream, various soups, beverages, alcoholic beverages, vitamin complex, etc., and the health functional food includes all health functional foods in the conventional sense.

The active ingredient of the present invention may be added to the food as it is or used with other foods or food ingredients and may be appropriately used according to a general method. The mixing amount of the active ingredients may be suitably determined according to the purpose of use thereof (for prevention or alleviation). In general, the active ingredient in the health functional food may be added in an amount of 0.1 to 90 parts by weight of the total food weight. However, in the case of long-term ingestion for the purpose of health and hygiene or health regulation, the amount may be the range or less and the active ingredient may be used even in the amount of the range or more.

A health functional beverage composition of the present invention is not particularly limited to ingredients other than containing the active ingredient as an essential ingredient in an indicated ratio and may contain various flavoring agents or natural carbohydrates as an additional ingredient, like conventional beverages. Examples of the above-described natural carbohydrates include general sugars, including monosaccharides, such as glucose, fructose, etc.; disaccharides, such as maltose, sucrose, etc.; and polysaccharides, such as dextrin, cyclodextrin, etc., and sugar alcohols such as xylitol, sorbitol, erythritol, etc. As flavoring agents other than those described above, natural flavoring agents (thaumatin, stevia extract (e.g., rebaudioside A, glycyrrhizin, etc.)) and synthetic flavoring agents (saccharin, aspartame, etc.) may be used. The ratio of the natural carbohydrates may be about 1 to 20 g, or about 5 to 12 g per 100 ml of the composition of the present invention. In addition, the active ingredients of the present invention may contain various nutrients, vitamins, minerals (electrolytes), flavoring agents such as synthetic and natural flavoring agents, coloring agents, and enhancers (cheese, chocolate, etc.), pectic acid and salts thereof, alginic acid and salts thereof, organic acid, a protective colloidal thickener, a pH adjusting agent, a stabilizer, a preservative, glycerin, alcohol, a carbonating agent used in a carbonated drink, etc. In addition, the active ingredient of the present invention may contain pulp for preparing natural fruit juice, fruit juice beverages, and vegetable beverages. These ingredients may be used independently or in combination.

As used herein, the term "feed" may mean any natural or artificial diet, one-meal diet, or ingredients of one-meal diet to be eaten, ingested, and digested by animals or suitable thereto. The type of feed is not particularly limited, and may use feeds commonly used in the art.

As used herein, the term "feed composition" may mean any natural or artificial diet, one-meal diet, or ingredients of the one-meal diet to be eaten, ingested, and digested by animals or suitable thereto. The type of feed composition is not particularly limited, and may mean a feed composition commonly used in the art. Non-limiting examples of the feed composition may include vegetable feeds, such as grains, root fruits, food processing by-products, algae, fibers, pharmaceutical by-products, oils and fats, starches, meals or grain by-products; and animal feeds such as proteins, inorganic materials, minerals, single-cell proteins, animal planktons, or foods. These feed compositions may be used alone or in combination of two or more kinds.

The feed composition of the present invention may include feed additives, and the *Paracoccus aminovorans* strain of the present invention, live cells thereof, dead cells thereof, a freeze-dried product thereof, a lysate thereof, an extract thereof, or a compound thereof may be a feed additive.

Furthermore, the feed composition of the present invention may further include excipients, diluents, and additives.

The *Paracoccus aminovorans* strain of the present invention is a *Paracoccus aminovorans* strain BM109 (Accession Number: KCTC15059BP) and may be used in various embodiments.

Hereinafter, the present invention will be described in more detail through Examples. However, these Examples are intended to illustrate one or more embodiments, and the scope of the present invention is not limited to these Examples.

### Example 1: Identification of TMA and TMAO degradation genes of Paracoccus aminovorans BM109 strain

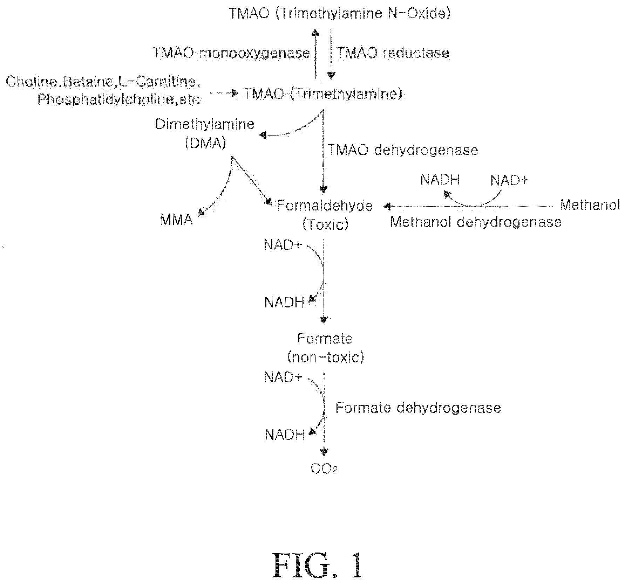

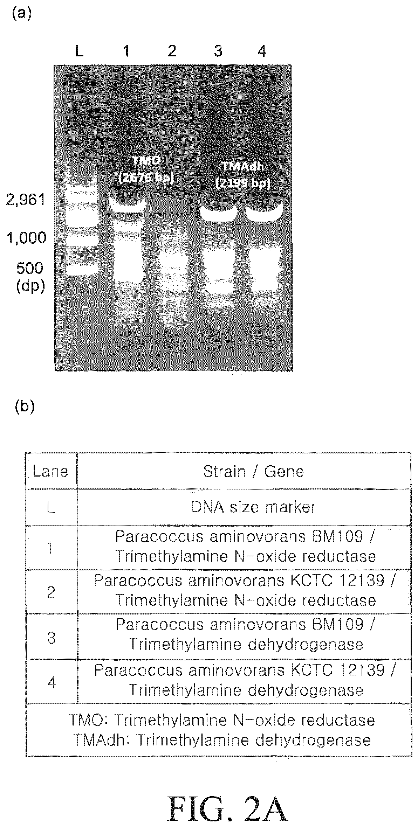

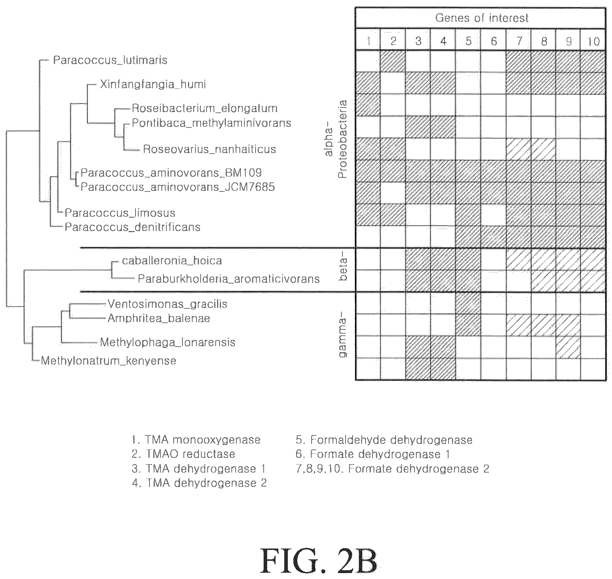

Hereinafter, with reference to FIGS. 1 and 2, TMA and TMAO degradation genes included in a *Paracoccus aminovorans* BM109 strain of the present invention will be described in detail.
FIG. 1 illustrates enzymes related to TMA and TMAO degradation of a *Paracoccus aminovorans* BM109 strain used in various embodiments of the present invention and a TMA and TMAO metabolic pathway using the enzymes.
FIG. 2 illustrates results of identifying enzyme genes related to TMA and TMAO degradation of a *Paracoccus aminovorans* BM109 strain used in various embodiments of the present invention.
FIG. 2A is a result of confirming through PCR and DNA electrophoresis that the *Paracoccus aminovorans* BM109 strain used in various embodiments of the present invention has a TMAO reductase gene.
FIG. 2B illustrates genome analysis results for confirming that the *Paracoccus aminovorans* BM109 strain used in various embodiments of the present invention has enzyme genes related to TMA and TMAO degradation.

First, referring to FIG. 1, the *Paracoccus aminovorans* BM109 strain of the present invention includes a TMA monooxygenase gene represented by SEQ ID NO: 1, a TMAO reductase gene represented by SEQ ID NO: 2, a TMA dehydrogenase 1 gene represented by SEQ ID NO: 3, a TMA dehydrogenase 2 gene represented by SEQ ID NO: 4, a formaldehyde dehydrogenase gene represented by SEQ ID NO: 5, a formate dehydrogenase 1 gene represented by SEQ ID NO: 6, and a formate dehydrogenase 2 gene represented by SEQ ID NO: 11, which is a set of four genes represented by SEQ ID NO: 7 to 10. Meanwhile, the *Paracoccus aminovorans* BM109 strain of the present invention has two types of TMA dehydrogenase and two types of formate dehydrogenase. Accordingly, the two types of TMA dehydrogenase genes were named a TMA dehydrogenase 1 gene and a TMA dehydrogenase 2 gene, and the two types of formate dehydrogenase genes were named a formate dehydrogenase 1 gene and a formate dehydrogenase 2 gene.

Meanwhile, referring to FIG. 2B together, the formate dehydrogenase 2 gene represented by SEQ ID NO: 11 consists of a set of four separate gene sequences represented by SEQ ID NOs: 7, 8, 9, and 10. Accordingly, the four separate gene sequences constituting the formate dehydrogenase 2 gene represented by SEQ ID NO: 11 were 7, 8, 9, and 10 in the 'Gene of interest' indicating the genes represented by SEQ ID NO: 1 to 10 in FIG. 2B.

Referring back to FIG. 1, the *Paracoccus aminovorans* BM109 strain of the present invention may convert TMAO into TMA through TMAO reductase, convert the TMA into dimethylamine (DMA) and formaldehyde through TMA dehydrogenase, convert the formaldehyde into formate, which is harmless to a subject, through formaldehyde dehydrogenase, and then convert the formate to carbon dioxide through formate dehydrogenase to finally complete-degrade the TMAO to carbon dioxide.

Meanwhile, referring to FIG. 2A, it was confirmed that the *Paracoccus aminovorans* BM109 strain of the present invention inherently contained the TMAO reductase gene represented by SEQ ID NO: 2, while a *Paracoccus aminovorans* KCTC12139 strain other than the present invention belonging to the same species did not contain the TMAO reductase gene.

FIG. 2A (a) illustrates a result of confirming through PCR and DNA electrophoresis the presence or absence of the TMAO reductase gene of each of the *Paracoccus aminovorans* BM109 strain of the present invention and the *Paracoccus aminovorans* KCTC12139 strain belonging to the same species as the strain of the present invention as a comparative group.

FIG. 2A (b) illustrates DNA fragment types of strains loaded into each of lanes of electrophoresis of FIG. 2A (a), that is, lane L and lanes 1 to 4.

First, referring to FIG. 2A (a), the TMAO reductase gene (TMO) was identified only in a *Paracoccus aminovorans* BM109 strain (Lane 1), and it was confirmed that *a Paracoccus aminovorans* KCTC 12139 strain (Lane 2) as a comparative group did not have the TMAO reductase gene.

As a result, it was confirmed that since the *Paracoccus aminovorans* KCTC 12139 strain (Lane 2) as the comparative group did not have the TMAO reductase gene unlike the *Paracoccus aminovorans* BM109 strain of the present invention, the TMAO could not be reduced to TMA, and thus the TMAO in the gut of the subject could not be degraded.

On the other hand, referring to FIG. 1 together, it was confirmed that the *Paracoccus aminovorans* BM109 strain of the present invention may convert TMAO to TMA through TMAO reductase to degrade TMA, thereby preventing the accumulation of TMAO.

Next, in the results of genome analysis to identify enzyme genes 1 to 5 related to TMA and TMAO degradation in FIG. 2B, it was analyzed that the *Paracoccus aminovorans* BM109 strain of the present invention had all of TMA monooxygenase, TMAO reductase, TMA dehydrogenase, formaldehyde dehydrogenase, and formate dehydrogenase.

In the genome analysis of FIG. 2B, if there was 75% or more homology based on 10 gene amino acid sequences represented by SEQ ID NOs. 1 to 10 for each microorganism shown in FIG. 2B, the microorganism was considered to have the gene, which was indicated in a dark shade on FIG. 2B. If the homology was low (75% or less), but there was a gene sequence analyzed as the corresponding protein in the base sequence annotation, it was indicated in a light shade.

Microorganisms subject to genome analysis were strains in allied species whose genome sequences were similar to the *Paracoccus aminovorans* BM109 strain of the present invention, and included microbial strains (*Paracoccus aminovorans* KCTC12139, *Paracoccus lutimaris*, *Paracoccus limosus*, *Paracoccus denitrificans, Roseibacterium elongatum*, *Roseovarius nanhaiticus*, *Xinfangfangia humi*, and *Pontibaca methylaminivorans*) of the *Alphaproteobacteria* order, Microbial strains (*Caballeronia choica*, *Paraburkholderia aromaticivorans*) of the *Betaproteobacteria* order of the *Proteobacteria* class, and microbial strains (*Methylophilus methylotrophus*, *Methylophaga lonarensis*, *Ventosimonas gracilis*, and *Amphritea balenae*) belonging to the *Gammaproteobacteria* order, to which the *Paracoccus aminovorans* BM109 strain of the present invention belonged.

Referring to FIG. 2B, as a result of genome sequence analysis, it was confirmed that only the *Paracoccus aminovorans* BM109 strain of the present invention had all 10 sequences (SEQ ID NOs: 1 to 10) of seven enzyme genes related to TMA and TMAO degradation.

On the other hand, it was confirmed that strains in allied species similar to the *Paracoccus aminovorans* BM109 strain of the present invention had only some of the genes related to TMA and TMAO degradation. In particular, it was confirmed that the *Paracoccus aminovorans* KCTC 12139 strain belonging to the same species as the present invention did not have the TMAO reductase gene, and the PCR and electrophoresis analysis results in FIG. 2A showed the same results. Accordingly, it was confirmed that only the *Paracoccus aminovorans* BM109 strain of the present invention had both genes related to TMA and TMAO degradation to convert TMAO into TMA through TMAO reductase to be completely degraded into carbon dioxide via formaldehyde and formate.

### Example 2: TMA and TMAO in vitro degradation ability tests of Paracoccus aminovorans BM109 strain

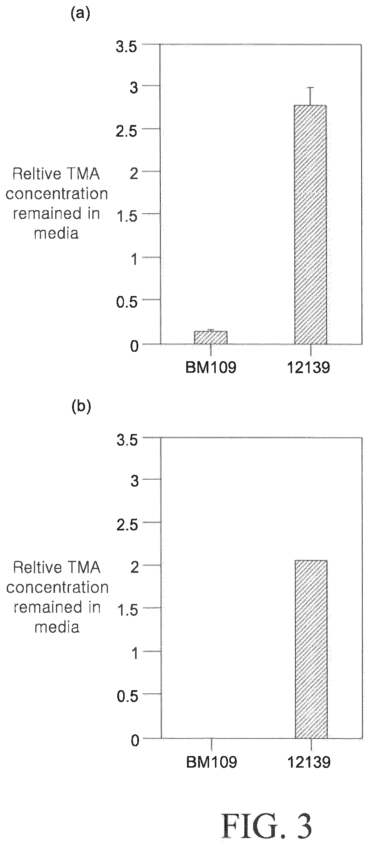

Hereinafter, referring to FIG. 3, TMA and TMAO *in vitro* degradation ability of *a Paracoccus aminovorans* BM109 strain according to various embodiments of the present invention will be described in detail.

FIG. 3 (a) illustrates the TMA removal ability of a *Paracoccus aminovorans* BM109 strain (BM109) and a *Paracoccus aminovorans* KCTC 12139 strain under *in vitro* aerobic culture conditions.

FIG. 3 (b) illustrates the TMA removal ability of a *Paracoccus aminovorans* BM109 strain (BM109) and a *Paracoccus aminovorans* KCTC 12139 strain (12139) under *in vitro* anaerobic culture conditions.

Referring to FIG. 3 (a), it was confirmed that the *Paracoccus aminovorans* BM109 strain of the present invention had about 30-fold higher TMA removal ability than the *Paracoccus aminovorans* KCTC 12139 strain as a comparative group. Furthermore, referring to FIG. 3 (b), it was confirmed that in the anaerobic conditions similar to the gut environment, the *Paracoccus aminovorans* BM109 strain of the present invention had at least 30-fold higher TMA removal ability than the *Paracoccus aminovorans* KCTC 12139 strain as a comparative group, and thus the *Paracoccus aminovorans* BM109 strain of the present invention had the excellent TMA removal effect in the gut.

Accordingly, as illustrated in FIG. 3, the *Paracoccus aminovorans* BM109 strain of the present invention has excellent removal ability of TMA, a precursor of TMAO, and may be usefully used to prevent accumulation of TMAO in the body.

### <TMA quantitative analysis>

Quantitative analysis of the concentration of TMA was performed using an enzyme immunoassay method using a TMA standard curve. First, in order to create a standard curve, bacteria were inoculated and cultured on a Luria-Bertani medium containing 5 mM TMA. A standard solution was prepared by diluting TMA in distilled water to 0, 1.25, 2.5, 5.0, and 10.0 mM, respectively. Thereafter, a liquid sample to be analyzed was centrifuged, 250 µL of the supernatant was added into a sterilized Eppendorf tube, 50 µL of 20% formaldehyde, 1 mL of toluene anhydride, and 150 µL of 50% potassium carbonate were added to the tube in sequence, and then the tube was vortexed for 10 seconds and shaken for 5 minutes, then placed in a tube rack and left to stand for 5 minutes. Thereafter, when the toluene layer was separated from the upper layer, 100 µL each of the toluene layer was loaded three times into a 96-well plate made of polypropylene. Absorbance was measured using an ELISA reader at a wavelength of 410 nm.

### Example 3: Identification of TMA and TMAO degradation ability in gut of Paracoccus aminovorans BM109 strain

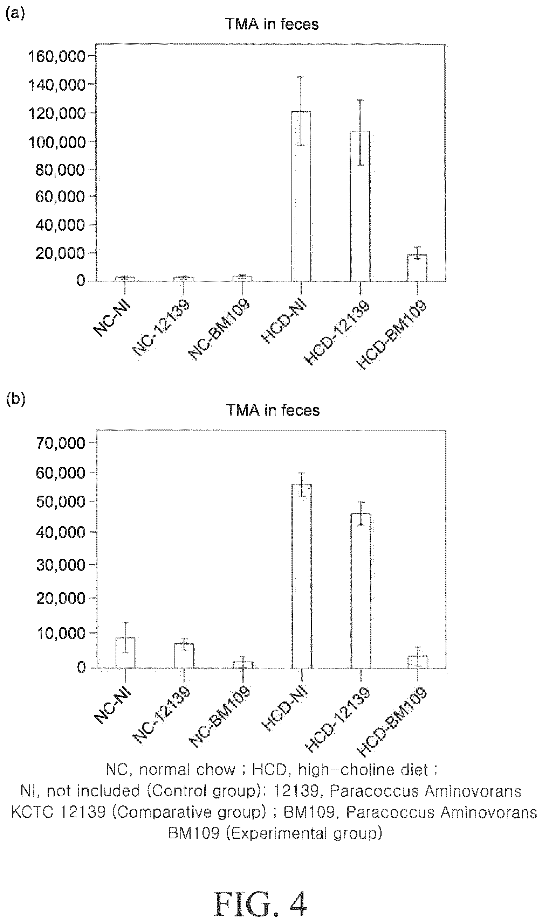

Hereinafter, referring to FIG. 4, *in vivo* TMA and TMAO degradation ability of *a Paracoccus aminovorans* BM109 strain according to various embodiments of the present invention will be described in detail.

FIG. 4 illustrates results of concentrations of TMA and TMAO in feces from *in vivo* tests for TMA and TMAO degradation ability of a *Paracoccus aminovorans* BM109 strain used in various embodiments of the present invention.

Specifically, FIG. 4 (a) illustrates the concentration of TMA in feces collected in an *in vivo* test for the TMA degradation ability of the *Paracoccus aminovorans* BM109 strain used in various embodiments of the present invention. Specifically, FIG. 4 (b) illustrates the concentration of TMAO in feces collected in an *in vivo* test for the TMA degradation ability of the *Paracoccus aminovorans* BM109 strain used in various embodiments of the present invention.

In Example, 5-week-old C57BL/6 mice were used as experimental animals, regardless of male and female. All mice were housed by 7 to 8 mice per cage and bred under conditions where the temperature of 22 to 24°C, humidity of 50 to 60%, and a 12-hour light/dark cycle were maintained. After an adaptation period of 1 week, all the mice were divided into two groups of normal chow (NC) and high choline diet (HCD) and bred for 45 weeks. During the breeding period, diet and water were consumed freely. At this time, the normal chow may be defined as a diet containing 0.08% of choline, and the high choline diet (HCD) may be defined as a diet containing 1% of choline.

The present inventors divided the mice bred on the normal chow (NC) or high-choline diet (HCD) for 45 weeks into three groups BM109, 12139, and NI, orally administered strains or phosphate buffer solution for 5 days for each group of a total of six groups, and then collected the feces on day 6. Thereafter, the feces were suspended in phosphate buffer solution at a ratio of 1 to 4 (w/v), centrifuged at 13,000 rpm for 5 minutes, and then the supernatant was taken, and the concentrations of TMA and TMAO were measured using mass spectrometry (LC-MS).

At this time, BM109 refers to a group administered orally by suspending the *Paracoccus aminovorans* BM109 strain in phosphate buffer solution at 10⁸ to 10¹² CFU/mL, 12139 refers to a group administered orally by suspending the *Paracoccus aminovorans* 12139 strain in phosphate buffer solution at 10⁸ to 10¹² CFU/mL, and NI refers to a group administered orally only with phosphate buffer solution in which the strain was not included (NI). The strain was suspended in phosphate buffer solution and cultured *in vitro* for 24 hours before oral administration.

As a result of the experiment, as shown in FIGS. 4A and 4B, the concentration of TMA in the feces of an HCD-NI group was 121,724 ng/mL (FIG. 4 (a)), and the concentration of TMAO in the feces of the HCD-NI group was 56,931 ng/mL (FIG. 4 (b)). Based on these results, it was confirmed that high concentrations of TMA and TMAO were accumulated in the gut of mice that consumed a high choline diet for a long period of time. On the other hand, when *the Paracoccus aminovorans* BM109 strain was orally administered for 5 days to mice that had consumed the high choline diet for a long period of time (FIG. 4 (a), HCD-BM109), it was confirmed that the concentration of TMA in the feces was 20,485 ng/mL, which was decreased to 16.8% compared to the HCD-NI group, and the concentration of TMAO was 3,673 ng/mL, which was decreased to 6.6% compared to the HCD-NI group. (FIG. 4 (b), HCD-BM109).

In addition, the TMA concentration in the feces of the HCD-BM109 group in FIG. 4 (a) was 20,485 ng/mL, which was 19.2% lower than the TMA concentration (106,854 ng/mL) of the HCD-12139 group. Furthermore, referring to FIG. 4 (b), the TMAO concentration in the feces of the HCD-BM109 group was 3,763.75 ng/mL, which was 7.88% lower than the TMAO concentration (47,754 ng/mL) of the HCD-12139 group. Based on the results, it was confirmed that the *Paracoccus aminovorans* BM109 strain of the present invention had excellent TMA and TMAO removal ability compared to the *Paracoccus aminovorans* 12139 strain.

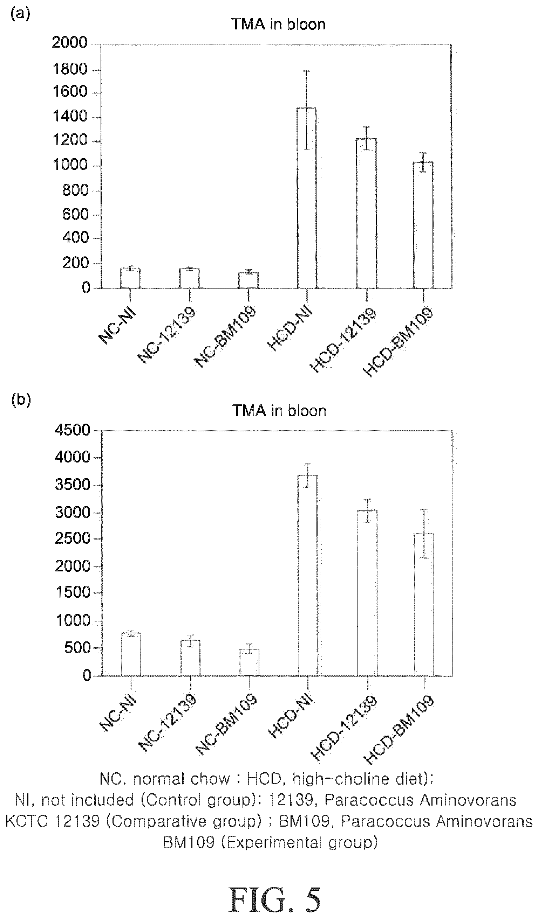

Further, the concentration of TMAO collected from the mouse feces of a normal chow group (NC-BM109) administered with the *Paracoccus aminovorans* BM109 strain of the present invention was 1,898.65 ng/mL, which was 19.7% to 25.6% lower than an NC-NI group (9,642 ng/mL) and an NC-12139 group (7,404.87 ng/mL), respectively (FIG. 5 (b)). In other words, it was confirmed that the *Paracoccus aminovorans* BM109 strain of the present invention may be useful in preventing various diseases caused by TMAO by showing an excellent effect in removing TMAO in feces even in the normal chow.

### Example 4: Identification of TMA and TMAO reduction effects in blood of Paracoccus aminovorans BM109 strain

Hereinafter, referring to FIG. 5, TMA and TMAO reduction effects in the blood of a *Paracoccus aminovorans* BM109 strain according to various embodiments of the present invention will be described in detail.

FIG. 5 illustrates concentration reduction effects of TMA and TMAO in the blood on TMA and TMAO degradation ability of a *Paracoccus aminovorans* BM109 strain used in various embodiments of the present invention.

*In vivo* Experimental Example to confirm the concentration reduction effects of TMA and TMAO in the blood on the TMA and TMAO degradation ability of the *Paracoccus aminovorans* BM109 strain of the present invention was the same as Example 3 above. In Example 3, the blood was collected at the same time as feces collection, plasma was separated from the blood, and TMA and TMAO concentrations were measured using mass spectrometry (LC-MS).

Referring to FIG. 5, FIG. 5 (a) illustrates an experimental result for a concentration reduction effect of TMA in the blood collected from mice on the TMA degradation ability of the *Paracoccus aminovorans* BM109 strain used in various embodiments of the present invention. In addition, FIG. 5 (b) illustrates an experimental result for a concentration reduction effect of TMAO in the blood collected from mice on the TMAO degradation ability of the *Paracoccus aminovorans* BM109 strain used in various embodiments of the present invention.

As a result of the experiment, as illustrated in FIGS. 5A and 5B, the concentration of TMA in the blood of an HCD-NI group was 1,473 ng/mL (FIG. 5 (a)), and the concentration of TMAO in the blood of the HCD-NI group was 3,666.3 ng/mL (FIG. 5 (b)). Based on these results, it was confirmed that high concentrations of TMA and TMAO were accumulated in the blood of mice that consumed a high choline diet for a long period of time. On the other hand, when the *Paracoccus aminovorans* BM109 strain of the present invention was orally administered for 5 days to mice that had consumed the high choline diet for a long period of time (FIG. 5 (a), HCD-BM109), it was confirmed that the concentration of TMA in the blood was 1045.9 ng/mL, which was decreased to 71% compared to the TMA concentration (1,473 ng/mL) of the HCD-NI group, and the concentration of TMAO was also 2,608.8 ng/mL, which was decreased to 71% compared to the TMAO concentration (3,666.3 ng/mL) of the HCD-NI group (FIG. 5 (b), HCD-BM109).

In addition, the TMA concentration in the blood of the HCD-BM109 group in FIG. 5 (a) was 1,045.9 ng/mL, which was 84.7% lower than the TMA concentration (1,233.87 ng/mL) of the HCD-12139 group. Furthermore, referring to FIG. 5 (b), the TMAO concentration in the blood of the HCD-BM109 group was 2,608.8 ng/mL, which was 86.7% lower than the TMAO concentration (3,008.42 ng/mL) of the HCD-12139 group. Based on these results, it was confirmed that the *Paracoccus aminovorans* BM109 strain of the present invention reduced the concentrations of TMA and TMAO in the gut, thereby also reducing the concentrations of TMA and TMAO in the blood.

Furthermore, the concentration of TMAO collected from the mouse blood of a normal chow group (NC-BM109) administered with the *Paracoccus aminovorans* BM109 strain of the present invention was 478.35 ng/mL, which was 61.7% to 72.4% lower than an NC-NI group (775.25 ng/mL) and an NC-12139 group (660.44 ng/mL), respectively (FIG. 5 (b)). In other words, it was confirmed that the *Paracoccus aminovorans* BM109 strain of the present invention can be useful in preventing various diseases caused by TMAO by showing an excellent effect of reducing TMAO in the blood even in the normal chow.

As the results shown in Examples 3 and 4, it was confirmed that the *Paracoccus aminovorans* BM109 strain of the present invention degraded TMAO to TMA by TMAO reductase in the gut and then degraded TMA, thereby degrading both the TMA and TMAO. In addition, it was confirmed that the *Paracoccus aminovorans* BM109 strain of the present invention was excellent in both TMA and TMAO removal abilities compared to the *Paracoccus aminovorans* KCTC 12139 strain without containing TMAO reductase.

In particular, it was confirmed that the *Paracoccus aminovorans* BM109 strain of the present invention was more effective in reducing TMAO in the feces of mice on the high choline diet, and thus can be more useful in preventing or treating various diseases caused by TMAO by removing effectively TMAO in the gut.

Although the embodiments of the present invention have been described in detail with reference to the accompanying drawings, the present invention is not limited thereto and may be embodied in many different forms without departing from the technical concept of the present invention. Therefore, the embodiments of the present invention are provided for illustrative purposes only but are not intended to limit the technical concept of the present invention. The scope of the technical concept of the present invention is not limited thereto. Therefore, it should be appreciated that the aforementioned embodiments are illustrative in all aspects and are not restricted. The protective scope of the present invention should be construed on the basis of the appended claims, and all the technical ideas in the equivalent scope thereof should be construed as falling within the scope of the present invention.
[National Research and Development Project Supporting Invention]
[Project Unique Number] 1425166770
[Project Number] S3287890
[Ministry Name] Ministry of SMEs and Startups (MSS)
[Project Management (Special) Institution Name] Korea Technology & Information Promotion Agency for SMEs
[Research Project Name] TECH INCUBATOR PROGRAM FOR STARTUP (TIPS)
[Research Subject Name] Development of Microbiome-based Microbial Therapeutic Product
[Percent Contribution] 1/1
[Project Performance Institute Name] BIOME Co., Ltd.
[Research Period] 20220501 to 20240430

## Claims

1. A *Paracoccus aminovorans* BM109 strain (Accession number KCTC 15059BP).

2. The *Paracoccus aminovorans* strain of claim 1, wherein the strain has TMA or TMAO degradation ability.

3. The *Paracoccus aminovorans* strain of claim 1, wherein the strain includes a trimethylamine N-oxide (TMAO) reductase gene represented by SEQ ID NO: 2.

4. The *Paracoccus aminovorans* strain of claim 3, wherein the strain further includes a trimethylamine (TMA) monooxygenase gene represented by SEQ ID NO: 1.

5. The *Paracoccus aminovorans* strain of claim 3, wherein the strain further includes a trimethylamine (TMA) dehydrogenase 1 gene represented by SEQ ID NO: 3.

6. The *Paracoccus aminovorans* strain of claim 3, wherein the strain further includes a TMA dehydrogenase 2 gene represented by SEQ ID NO: 4.

7. The *Paracoccus aminovorans* strain of claim 3, wherein the strain further includes a formaldehyde dehydrogenase gene represented by SEQ ID NO: 5.

8. The *Paracoccus aminovorans* strain of claim 3, wherein the strain further includes a formate dehydrogenase 1 gene represented by SEQ ID NO: 6.

9. The *Paracoccus aminovorans* strain of claim 3, wherein the strain further includes a formate dehydrogenase 2 gene represented by SEQ ID NO: 11.

10. The *Paracoccus aminovorans* strain of claim 9, wherein the formate dehydrogenase 2 gene represented by SEQ ID NO: 11 consists of a set of four separate gene sequences represented by SEQ ID NOs: 7, 8, 9 and 10.

11. The *Paracoccus aminovorans* strain of claim 2, **characterized in that** the strain includes a TMAO reductase gene that converts trimethylamine N-oxide (TMAO) to trimethylamine (TMA), thereby having further increased TMA or TMAO removal ability, compared to *Paracoccus aminovorans* strains without the TMAO reductase.

12. The *Paracoccus aminovorans* strain of claim 1, wherein the strain has increased trimethylamine removal ability under anaerobic conditions.

13. The *Paracoccus aminovorans* strain of claim 12, wherein the anaerobic conditions have an oxygen concentration of 3 to 10%.

14. A pharmaceutical composition for preventing or treating diseases caused by TMA or TMAO comprising a *Paracoccus aminovorans* strain, live cells thereof, dead cells thereof, a freeze-dried product thereof, a lysate thereof, an extract thereof, or a compound thereof.

15. The pharmaceutical composition of claim 14, wherein the disease caused by the TMA or TMAO is a metabolic disease including trimethylaminuria or fish odor syndrome.

16. The pharmaceutical composition of claim 14, wherein the disease caused by the TMA or TMAO is a disease caused by accumulation of the TMAO, and the disease is a cardiovascular disease including at least one selected from atherosclerosis, severe heart failure, coronary heart disease, inflammatory heart disease, valvular heart disease, stroke, thrombosis, myocardial infarction, and reactive thrombocytosis.

17. The pharmaceutical composition of claim 14, wherein the disease caused by the TMA or TMAO is a disease caused by accumulation of the TMAO, and
the disease is a kidney disease including at least one selected from renal failure, chronic kidney disease, polycystic kidney disease, glomerulonephritis, IgA nephropathy, and kidney syndrome.

18. A health functional food composition for preventing or improving diseases caused by TMA or TMAO, comprising a *Paracoccus aminovorans* strain, live cells thereof, dead cells thereof, a freeze-dried product thereof, a lysate thereof, an extract thereof, or a compound thereof.

19. A feed composition for preventing or improving diseases caused by TMA or TMAO, comprising a *Paracoccus aminovorans* strain, live cells thereof, dead cells thereof, a freeze-dried product thereof, a lysate thereof, an extract thereof, or a compound thereof.
